# FASCICULE DE BREVET EUROPEEN

(11) **EP 0 955 283 B1**
(45) Date de publication et mention de la délivrance du brevet: **18.09.2002**
(21) Numéro de dépôt: 99400766.4
(22) Date de dépôt: 30.03.1999
(51) Int. Cl.: C07C 17/14, C07C 22/04, C07C 22/06

(54) **Procédé de préparation en continu de mono et/ou bis(mono-, et/ou di-, et/ou trichlorométhyl) benzènes**
Verfahren zur kontinuierlichen Herstellung von Mono und/oder Bis(Mono-, und/oder Di-, und/oder Trichlormethyl)benzolen
Process for the continuous preparation of mono and/or bis(mono-, and/or di-, and/or trichloromethyl)benzenes

(30) Priorité: 09.04.1998 FR 9804460
(43) Date de publication de la demande: 10.11.1999
(73) Titulaire: Tessenderlo Chemie N.V., 3980 Tessenderlo (BE)
(72) Inventeur: Commandeur, Raymond, 38220 Vizille (FR); Loze, Régis, 04600 Saint-Auban (FR)
(74) Mandataire: Brants, Johan Philippe Emile

(56) Documents cités:
- FR-A- 2 156 911
- GB-A- 583 634
- GB-A- 1 401 038
- US-A- 2 810 688
- US-A- 4 056 455

## Description

La présente invention concerne un procédé continu de préparation de mono- et/ou bis-(mono-, et/ou di- et/ou trichlorométhyl) benzènes.

Les mono- et/ou bis-(mono-, et/ou di- et/ou trichlorométhyl) benzènes sont des intermédiaires importants pour la préparation d'agents pesticides, de colorants, d'herbicides et des additifs pour les matières plastiques (anti-UV).

La technique habituelle pour obtenir ces intermédiaires chlorés consiste à effectuer la chloration des méthylbenzènes initiée chimiquement ou photochimiquement.

Cette réaction est effectuée industriellement généralement avec du chlore gazeux. Cette réaction étant celle d'un gaz avec un liquide, la cinétique globale de la réaction est régie à la fois par la cinétique chimique et la cinétique physique, c'est-à-dire la diffusion du chlore gazeux. La cinétique chimique dépend essentiellement de la nature du ou des substituants sur le benzène ainsi que de la méthode d'initiation.

Il s'agit d'une réaction radicalaire en chaîne, le rendement diminuant nettement entre les différents stades de la chloration, c'est-à-dire du -CH₃ de départ au composé monochloré -CH₂Cl puis au composé dichloré -CHCl₂, et enfin au composé trichloré -CCl₃.

Aussi, vers la fin de la réaction, lorsque la teneur en atomes d'hydrogène sur le -CH₃ susceptible d'être substitué est relativement faible, des réactions secondaires, telles que chloration sur le noyau sont susceptibles de se produire.

Notamment, ces réactions secondaires seront d'autant plus importantes que la quantité de chlore disponible sera élevée et lorsque les espèces radicalaires ne seront plus présentes en quantité suffisante.

Afin de pallier à ces inconvénients, on propose dans la demande de brevet FR 2156911 relative à la préparation du trichlorométhylbenzène de diluer le chlore en excès avec un gaz inerte tels que l'azote afin de diminuer les réactions secondaires indésirables.

Egalement, dans cette demande, on utilise des temps de séjours (temps de contact) importants pour atteindre la chloration complète des intermédiaires sous-chlorés.

Cependant, cette façon d'opérer présente l'inconvénient qu'une quantité de chlore trop importante passe nécessairement dans les effluents, avec le gaz inerte, ce qui nécessite des opérations continues de traitement desdits effluents, dans l'éventualité où l'on désire récupérer le gaz chlorhydrique produit lors de la réaction ; ou bien des opérations de destruction, ou bien encore, comme dans la demande précitée d'utiliser une seconde installation de chloration. En effet, dans cette demande FR 2156911 on effectue la chloration photochimique du toluène dans une cascade de réacteurs divisée en deux sections.

Dans une première section constituée de 5 réacteurs, on injecte 2,3 à 2,9 moles de chlore par mole de toluène, soit 86 à 96 % de la quantité de chlore nécessaire théorique pour préparer le trichloromethylbenzène.

Ensuite, on chlore le mélange obtenu dans la seconde section, constituée de 4 réacteurs, avec un excès de chlore dilué par un gaz inerte.

Les effluents des derniers réacteurs passent dans le premier réacteur de la première section. La teneur en chlore de l'HCl sortant est inférieure à 1 %.

Dans la seconde section, le dernier réacteur a un volume qui représente 2 à 3 fois le volume de tous les réacteurs précédents des deux sections.

Cependant, ce dispositif ne permet pas de supprimer d'une façon suffisante les produits secondaires. Le produit final obtenu contient environ 5 % de produits secondaires chlorés dans le noyau.

Dans le brevet US 4 056 455, on décrit un procédé continu de photochloration du toluène qui consiste à opérer dans une cascade de 10 réacteurs.

Le toluène est introduit dans le premier réacteur, les réacteurs 2 à 9 étant alimentés par des quantités déterminées de chlore gazeux.

Les effluents des réacteurs 5 à 10, riches en chlore, retournent aux réacteurs 2 et 3.

De même, les effluents des réacteurs 2 à 4 sont introduits dans le réacteur 1 contenant une forte concentration en toluène de sorte qu'une grande partie du chlore contenu dans l'HCl est transformé.

Cependant, avec ce procédé, on constate que le gaz chlorhydrique contient encore une quantité pondérale de chlore importante qui est encore au plus égale à 2 %.

Cette quantité de chlore conduit inévitablement à la chloration sur le noyau des méthylbenzènes entraînés lors de l'abattage de chlorure d'hydrogène par l'eau. Ceci conduit à une mauvaise qualité des solutions d'acide chlorhydrique car il est difficile d'éliminer par stripping les méthylchlorobenzènes (produits lourds) et pratiquement impossible de recycler la phase les contenant.

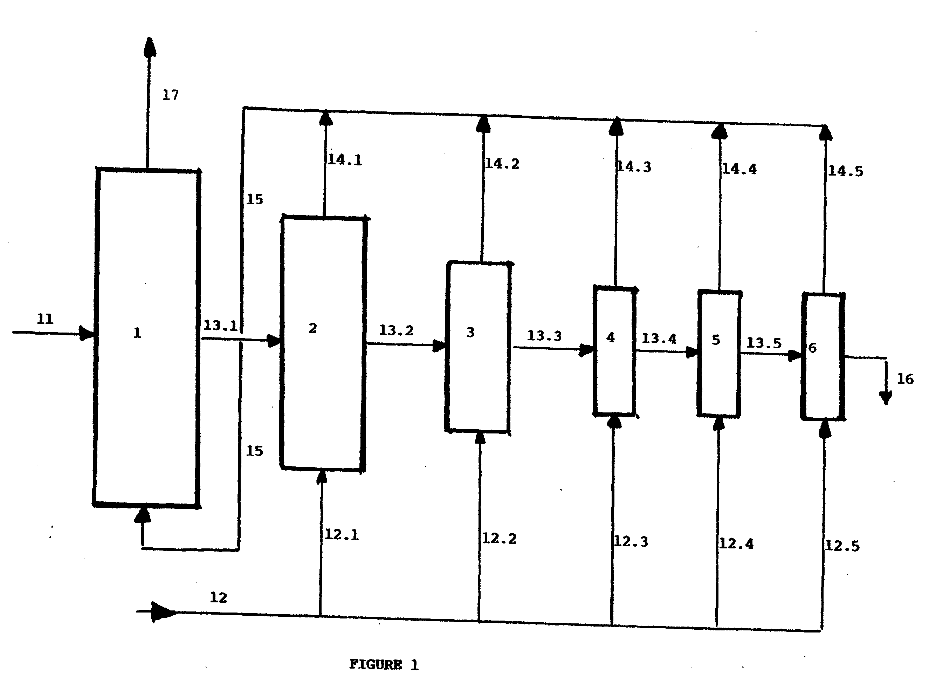

Par ailleurs, on constate que, sur la figure 1 schématisant l'installation, les 10 réacteurs sont représentés de la même façon. Aussi, il n'y a rien qui puisse laisser supposer que lesdits réacteurs seraient de taille différente.

Il est par ailleurs généralement admis que l'oxygène a un effet sur les réactions radicalaires notamment sur les réactions de chloration des composés organiques aromatiques ou aliphatiques. Cet effet pouvant être favorable ou défavorable à l'avancement de la réaction.

Ainsi, Serguchev Jv. A. et col. (Zhurnal org. khim ; (1983) XIX vol. 5, pages 1020-1023) ont étudié l'action de l'oxygène sur la vitesse de chloration radicalaire du toluène au niveau de la chaîne latérale.

Ils ont montré qu'en opérant avec du toluène soigneusement désoxygéné à des températures comprises entre 90°C et 130°C ; et en utilisant du chlore avec une teneur en O₂ voisine de 0,02 % en volume, la chloration du toluène en trichlorométhylbenzène s'effectuait avec un rendement de 95 % au bout de 26 heures en l'absence d'initiateurs chimiques et de radiation lumineuse.

On a maintenant trouvé un procédé continu de préparation de mono- et/ou bis(mono-, et/ou di- et/ou trichlorométhyl)benzènes, éventuellement substitués sur le cycle benzènique par un ou plusieurs atomes d'halogène, par chloration progressive des mono- ou diméthylbenzènes correspondants dans (au moins deux) plusieurs réacteurs en cascade, caractérisé en ce que le volume du premier réacteur est 1,5 fois à 2 fois le volume du second réacteur, le cas échéant 2,5 à 3 fois le volume du troisième réacteur, 8 à 10 fois le volume de chacun des autres réacteurs et que l'on amène audit premier réacteur la totalité des gaz résiduaires des autres réacteurs.

Selon la présente invention, on opère sous irradiation lumineuse, à une température comprise entre 50°C et 180°C et, de préférence, comprise entre 75°C et 145°C.

On opère également en présence d'un quantité d'oxygène au plus égale à 50 vpm et, de préférence, au plus égale à 20 vpm dans les gaz (chlore plus HCI).

Selon la présente invention, les mono- ou diméthylbenzènes introduits dans le premier réacteur sont préalablement déoxygénés par séchage azéotropique ou (de préférence) par stripping par les effluents gazeux constitués en majorité par le chlorure d'hydrogène formé et sortant dudit premier réacteur.

Le volume du premier réacteur est supérieur au volume du ou des autres réacteurs.

Le chlore gazeux est purifié par les moyens classiques connus de l'homme du métier tels que le dégazage. On pourra utiliser aussi préférentiellement du chlore revaporisé provenant d'un procédé de fabrication conduisant à un chlore contenant peu d'oxygène tel que le procédé d'électrolyse dit à membrane.

Selon la présente invention, le volume du premier réacteur est 1,5 fois à 2 fois le volume du second réacteur, le cas échéant, 2,5 fois à 3 fois le volume du troisième réacteur, et 8 à 10 fois le volume de chacun des autres réacteurs.

Selon la présente invention, le nombre de réacteurs varie dans une large mesure et dépend notamment du degré de chloration souhaité et des spécifications exigées pour les produits finis.

Si l'on souhaite obtenir les mono- ou bis-(trichlorométhyl)benzènes on utilisera un nombre de réacteurs au moins égal à 5 et, de préférence, égal à 6. Dans cette configuration, on alimente en chlore frais tous les réacteurs à l'exception du premier.

Dans le cas où l'on souhaite obtenir les mono- ou bis-(mono, ou dichlorométhyl)benzènes on utilisera 2 voir 3 réacteurs. Dans cette configuration, tous les réacteurs peuvent être alimentés en chlore gazeux frais.

Selon la présente invention, on utilise comme source d'irradiation lumineuse des lampes à vapeur de mercure de puissance comprise entre 1 et 15 kW et, de préférence, comprise entre 3 et 10 kW.

Le nombre de ces lampes par réacteur peut varier dans une large mesure. Il est fonction de la puissance désirée. Ce nombre peut aller de 1 à 5 par réacteur. Ces lampes sont de préférence immergées dans le milieu réactionnel.

Selon la présente invention, le ou les premiers réacteurs sont des réacteurs classiques comprenant éventuellement des chicanes dont l'agitation est réalisée par le courant gazeux. Les suivants et derniers réacteurs sont de préférence des réacteurs de type piston.

Les réacteurs sont équipés d'au moins une lampe axiale supportée en partie haute et traversant presque entièrement le réacteur. Le liquide et le chlore sont introduits en partie basse et remontent vers la partie haute du réacteur dont ils sont extraits.

La chaleur de réaction dégagée par la chloration peut être évacuée des réacteurs par tout moyen connu tel que courant continu d'eau froide circulant dans la double-enveloppe ou bien par un échangeur situé à l'intérieur ou à l'extérieur des réacteurs ou bien encore par vaporisation des composés les plus volatiles.

On opère généralement de préférence à pression atmosphérique. Des pressions plus hautes ou plus basses peuvent être utilisées mais ne confèrent généralement aucune amélioration sensible au procédé. De préférence, on choisira une pression de façon à ce que le chlore se trouve toujours sous forme gazeuse aux températures utilisées dans chaque réacteur.

Selon la présente invention, les températures, identiques ou différentes pour chaque réacteur, sont comprises entre 50°C et 180°C et, de préférence, comprises entre 75°C et 145°C.

Selon la présente invention, le mélange réactionnel de chloration liquide passe avantageusement d'un réacteur à l'autre par gravité et est amené, après le dernier réacteur dans une cuve de stockage.

Les gaz résiduaires de tous les réacteurs à l'exception du premier réacteur, comprenant du chlorure d'hydrogène formé, du chlore non réagi et éventuellement des produits chlorés entraînés, sont introduits dans le premier réacteur.

L'acide chlorhydrique sort de ce premier réacteur avec une teneur pondérale en chlore généralement inférieure à 0,2 %.

Le rapport molaire chlore/composé à chlorer est fonction du (ou des) produit(s) chloré(s) souhaité(s).

Ainsi, ce rapport est égal à environ 3/1 lorsque l'on désire obtenir le trichlorométhylbenzène.

Dans le cas où l'on désire obtenir préférentiellement des dichlorométhylbenzènes, ce rapport sera supérieur à 2/1 et, de préférence, compris entre 2,01/1 et 2,05/1.

Dans le cas où l'on désire obtenir un mélange de mono- et (dichlorométhyl)benzènes, ce rapport variera dans une large mesure et sera fonction des quantités de mono- et (dichlorométhyl)benzènes souhaités.

Dans le cas où les produits de réaction sont solides comme par exemple le 1,4-bis(trichlorométhyl)benzène, on peut utiliser des solvants légers inertes choisis de préférence dans le groupe des solvants fluorés ou chlorés. A titre d'illustration de tels solvants, on citera le tétrachlorure de carbone, le chloroforme, le 1,4-chlorotrifluorométhylbenzène.

Ainsi, si l'on souhaite obtenir un mélange riche en chlorure de benzyle, on opérera avantageusement avec un rapport molaire chlore sur toluène compris entre 0,4 et 0,6.

A titre d'illustration de mono- ou diméthylbenzènes utilisables selon la présente invention, on citera le toluène, les chlorotoluènes, les fluorotoluènes, les dichloro 2,4- et 2,6 toluènes, les xylènes et le mélange d'au moins deux des composés précités.

Le procédé s'applique tout particulièrement au toluène, aux xylènes et au parachlorotoluène.

Ce procédé présente l'avantage de conduire à un rendement élevé en mono- et/ou bis(mono-, et/ou di- et/ou trichlorométhyl)benzènes

En outre, l'HCl obtenu a une teneur pondérale en chlore ≤ 0,2 %.

Ce procédé présente également une grande flexibilité puisque en modulant le nombre de réacteurs on peut produire des mono- ou bis-(trichlorométhyl) benzènes ou bien des mono- ou bis(mono- et/ou dichlorométhyl)benzènes.

Les exemples qui suivent illustrent l'invention.

### EXEMPLE 1

### PREPARATION DU TRICHLOROMETHYLBENZENE PAR CHLORATION DU TOLUENE.

On utilise une installation consistant en 6 réacteurs émaillés en cascade tel que représentée sur la figure 1, lesdits réacteurs étant équipés de lampes à vapeur de mercure, immergées dans le milieu réactionnel (non représentées sur la figure 1).

Les volumes des réacteurs, le nombre de lampes par réacteur et la puissance de ces lampes sont indiqués dans le tableau 1. Dans cette installation, on fait réagir par heure 6,82 kilomoles de toluène et 20,7 kilomoles de chlore frais en continu, ce qui correspond à un rapport molaire chlore sur toluène égale à 3,03.

Les températures de chaque réacteur sont reportées dans le tableau 1.

On effectue l'introduction du chlore frais dans les réacteurs 2 à 6 respectivement par les conduites 12.1, 12.2, 12.3, 12.4, 12.5 à des débits horaires tels qu'indiqués dans le tableau 1.

Le toluène, préalablement désoxygéné et ayant une teneur en oxygène inférieure à 5 mg/kg, est amené au réacteur 1 par la conduite 11 à un débit de 627,44 kg/h puis traverse les réacteurs 2 à 6 par gravité respectivement par les conduites 13.1, 13.2, 13.3, 13.4, 13.5 tout en formant du C₆H₅CCl₃.

**TABLEAU 1**

| | | **LAMPE A VAPEUR DE MERCURE** | | **TEMPERATURE (°C)** | **ALIMENTATION EN** | |
|---|---|---|---|---|---|---|
| **REACTEUR** | **VOLUME (m**^{**3**}**)** | **NOMBRE PAR REACTEUR** | **PUISSANCE DE CHAQUE LAMPE (W)** | | **TOLUENE (kg/h)** | **CHLORE FRAIS (kg/h)** |
| 1 | 2,5 | 3 | 4 | 96,1 | 627,44 | - |
| 2 | 1,5 | 3 | 4 | 151,8 | - | 783,7 |
| 3 | 1 | 3 | 4 | 135,9 | - | 392,9 |
| 4 | 0,3 | 1 | 8 | 144,3 | - | 224,9 |
| 5 | 0,3 | 1 | 8 | 143,5 | - | 57,9 |
| 6 | 0,3 | 1 | 8 | 138,1 | - | 11,1 |

Par la conduite 16 on obtient par heure 1335,48 kg de trichlorométhylbenzène.

Dans le tableau 1, on indique également le débit d'alimentation du réacteur 1 en toluène et les débits d'alimentation des réacteurs 2 à 6 en chlore frais.

Les gaz résiduaires sortant des réacteurs 2 à 6, respectivement par les conduites 14.1, 14.2, 14.3, 14.4 et 14.5 sont colléctés dans la conduite 15 et sont introduits dans le réacteur 1. Ces gaz résiduaires entrant dans le réacteur 1 contiennent des quantités pondérales en HCI gaz et en chlore non réagi respectivement égales à environ 40 % et à environ 13 %. Ils contiennent également du toluène non transformé (environ 8 %) et des mono- di et trichlorométhylbenzènes entraînés.

Dans le tableau 2, nous avons indiqué la composition pondérale des produits liquides de réaction sortant de chaque réacteur. Dans ce tableau : Y1 représente le chlorométhylbenzène, Y2 représente le dichlorométhylbenzène, Y3 représente le trichlorométhylbenzène.

Par lourds, on désigne les produits chlorés sur le noyau.

Le chlorure d'hydrogène sortant du réacteur 1 par la conduite 17 a une teneur pondérale en chlore inférieure à 0,2 %.

Le trichlorométhylbenzène obtenu a une pureté de 97,7 % ; la teneur pondérale en dichlorométhylbenzène est de 0,11 %, le reste des sous-produits étant constitué essentiellement par des trichlorométhylbenzènes chlorés sur le noyau ou chlorés additivement sur les doubles liaisons ainsi que des produits de couplage à deux cycles aromatiques.

**TABLEAU 2**

| **REACTEUR** | **COMPOSITION DES PRODUITS LIQUIDES SORTANT DE CHAQUE REACTEUR (% POIDS)** | | | | |
|---|---|---|---|---|---|
| | **TOLUENE** | **Y1** | **Y2** | **Y3** | **LOURDS** |
| 1 | 38,23 | 23,11 | 16,22 | 20,54 | 0,16 |
| 2 | 2,3 | 17,78 | 41,71 | 36,72 | 0,28 |
| 3 | 0,03 | 2,15 | 27,60 | 68,95 | 0,61 |
| 4 | 0,01 | 0,75 | 11,99 | 86,05 | 0,56 |
| 5 | - | - | 0,73 | 97,36 | 0,93 |
| 6 | - | - | 0,11 | 97,74 | 1,22 |

### EXEMPLE 2

### PREPARATION DU CHLOROMETHYLBENZENE ET DU DICHLOROMETHYLBENZENE.

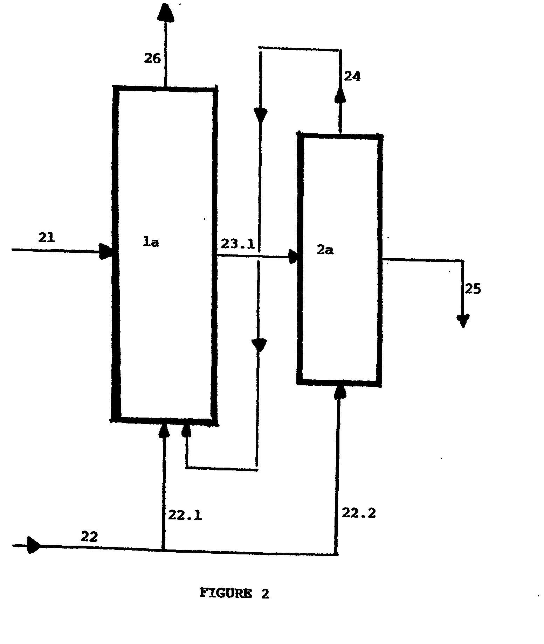

On utilise une installation consistant en 2 réacteurs émaillés en cascade tel que représenté sur la figure 2, lesdits réacteurs étant équipés de lampes à vapeur de mercure, immergées dans le milieu réactionnel (non représentées sur la figure 2). Les volumes des deux réacteurs, le nombre de lampes par réacteur et la puissance de ces lampes sont indiqués dans le tableau 3.

Dans cette installation, on fait réagir par heure 33,15 kilomoles de toluène et 18,31 kilomoles de chlore frais en continu, ce qui correspond à un rapport molaire chlore frais sur toluène égal à 0,55.

Les températures des deux réacteurs sont indiquées dans le tableau 3.

On effectue l'introduction du chlore frais dans les réacteurs la et 2a respectivement par les conduites 22.1 et 22.2 à des débits horaires tels qu'indiqués dans le tableau 3.

Le toluène, préalablement désoxygéné et ayant une teneur en oxygène égale à environ 3 mg/kg est amené au réacteur 1a par la conduite 21 à un débit de 3050 kg/h puis entre dans le réacteur 2a par gravité par la conduite 23.1 tout en formant un mélange de C₆H₅CH₂Cl et C₆H₅CHCl₂.

Par la conduite 25, on obtient par heure 3681,7 kg d'un mélange comprenant, en poids, 51,93 % de chlorométhylbenzène, 6,78 % de dichlorométhylbenzène et 41,12 % de toluène non transformé. Ce mélange est ensuite soumis à une distillation fractionnée sous pression réduite.

**TABLEAU 3**

| | | **Lampe à vapeur de mercure** | | | |
|---|---|---|---|---|---|
| **Réacteur** | **Volume (m**^{**3**}**)** | **Nombre par réacteur** | **Puissance de chaque lampe (W)** | **Température (°C)** | **Alimentation en chlore frais (kg/h)** |
| 1/a | 2,5 | 3 | 4 | 100 | 767,1 |
| 2/a | 1,5 | 3 | 4 | 116,6 | 533,5 |

Les gaz résiduaires sortant du réacteur 2a par la conduite 24 sont introduits dans le réacteur 1a. Ces gaz contiennnent des quantités pondérales en HClgaz et en chlore non réagi respectivement égales à environ 42 % et inférieur à 1 %.

Dans le tableau 4, nous avons indiqué la composition pondérale des produits liquides de réaction sortant de chaque réacteur.

Dans ce tableau, Y1 représente le chlorométhylbenzène, Y2 représente le dichlorométhylbenzène, Y3 le trichlorométhylbenzène.

**TABLEAU 4**

| **REACTEUR** | **COMPOSITION DES PRODUITS LIQUIDES SORTANT DE CHAQUE REACTEUR (% EN POIDS)** | | | |
|---|---|---|---|---|
| | **Toluène** | **Y1** | **Y2** | **Y3** |
| 1/a | 61,84 | 35,09 | 3. | 0,05 |
| 2/a | 41,12 | 51,93 | 6,78 | 0,17 |

Le chlorure d'hydrogène sortant du réacteur 1a par la conduite 26 a une teneur pondérale en chlore inférieure à 0,2 %.

## Revendications

1. Procédé continu de préparation de mono- et/ou bis(mono-, et/ou di et/ou trichlorométhyl)benzènes, éventuellement substitués sur le cycle benzénique par un ou plusieurs atomes d'halogène, par chloration progressive des mono- ou diméthylbenzènes correspondants dans plusieurs réacteurs en cascade, **caractérisé en ce que** le volume du premier réacteur est 1,5 fois à 2 fois le volume du second réacteur, le cas échéant 2,5 à 3 fois le volume du troisième réacteur, 8 à 10 fois le volume de chacun des autres réacteurs et que l'on amène audit premier réacteur la totalité des gaz résiduaires sortant des autres réacteurs.

2. Procédé selon la revendication 1, **caractérisé en ce que** l'on opère sous irradiation lumineuse.

3. Procédé selon l'une des revendications 1 ou 2, **caractérisé en ce que** l'on opère à une température comprise entre 50°C et 180°C.

4. Procédé selon l'une des revendications 1 à 3, **caractérisé en ce que** l'on opère en présence d'une quantité d'oxygène au plus égalé à 50 vpm dans les gaz.

5. Procédé continu de préparation de trichlorométhylbenzène selon l'une des revendications 1 à 4, **caractérisé en ce que** l'on opère dans 6 réacteurs en cascade.

6. Procédé selon la revendication 5, **caractérisé en ce que** l'on opère en introduisant du chlore frais dans les réacteurs à l'exception du premier réacteur.

7. Procédé continu de préparation de (mono- et dichlorométhyl)benzènes selon l'une des revendications 1 à 4, **caractérisé en ce que** l'on opère dans 2 réacteurs.

8. Procédé selon la revendication 7, **caractérisé en ce que** l'on opère en introduisant du chlore frais dans les 2 réacteurs.

## Patentansprüche

1. Kontinuierliches Verfahren zur Zubereitung von Monound/oder Bis-(Mono-, und/oder Di- und/oder Trichlormethyl)benzenen, die eventuell auf dem Benzenzyklus durch ein oder mehrere Halogenatome durch progressive Chlorierung der entsprechenden Mono- oder Dimethylbenzene in mehreren in Kaskade geschalteten Reaktoren substituiert sind, **dadurch gekennzeichnet, daß** das Volumen des ersten Reaktors 1,5 bis 2 mal so groß wie das Volumen des zweiten Reaktors, gegebenenfalls 2,5 bis 3 mal so groß wie das Volumen des dritten Reaktors, 8 bis 10 mal so groß wie das Volumen jedes der weiteren Reaktoren ist, und daß dem ersten Reaktor die Gesamtheit der aus den anderen Reaktoren austretenden Restgase zugeführt wird.

2. Verfahren nach Anspruch 1, **dadurch gekennzeichnet, daß** unter Lichteinstrahlung vorgegangen wird.

3. Verfahren nach einem der Ansprüche 1 oder 2, **dadurch gekennzeichnet, daß** bei einer Temperatur zwischen 50 °C und 180 °C vorgegangen wird.

4. Verfahren nach einem der Ansprüche 1 bis 3, **dadurch gekennzeichnet, daß** im Beisein einer Sauerstoffmenge von höchstens gleich 50 vpm in den Gasen vorgegangen wird.

5. Kontinuierliches Verfahren zur Zubereitung von Trichlormethylbenzen nach einem der Ansprüche 1 bis 4, **dadurch gekennzeichnet, daß** in 6 in Kaskade geschalteten Reaktoren vorgegangen wird.

6. Verfahren nach Anspruch 5, **dadurch gekennzeichnet, daß** durch Einleitung von frischem Chlor in die Reaktoren mit Ausnahme des ersten Reaktors vorgegangen wird.

7. Kontinuierliches Verfahren zur Zubereitung von (Monound Dichlormethyl)benzenen nach einem der Ansprüche 1 bis 4, **dadurch gekennzeichnet, daß** in 2 Reaktoren vorgegangen wird.

8. Verfahren nach Anspruch 7, **dadurch gekennzeichnet, daß** durch Einleitung von frischem Chlor in 2 Reaktoren vorgegangen wird.

## Claims

1. A continuous method for preparing mono- and/or bis(mono-, and/or di- and/or trichloromethyl) benzenes, possibly substituted on the benzene ring by one or more halogen atoms, by progressive chlorination of the corresponding mono- or dimethylbenzenes in several cascaded reaction vessels, **characterised in that** the volume of the first reaction vessel is 1.5 times to 2 times the volume of the second reaction vessel, and if such exist, 2.5 to 3 times the volume of the third reaction vessel, and 8 to 10 times the volume of each of the other reaction vessels and that the whole of the residual gases issuing from the subsequent reaction vessels is recycled to the first reaction vessel.

2. The method according to the claim 1, **characterised in that** it takes place under conditions of light irradiation.

3. The method according to either of claims 1 or 2, **characterised in that** it takes place at a temperature between 50 °C and 180 °C.

4. The method according to any of claims 1 to 3, **characterised in that** it takes place in the presence of a quantity of oxygen equal to no more than 50 parts per million by volume in the gases.

5. The continuous method for preparing trichloromethylbenzene according to any of claims 1 to 4, **characterised in that** it takes place in 6 cascaded reaction vessels.

6. The method according to claim 5, **characterised in that** it takes place with the introduction of fresh chlorine into the reaction vessels with the exception of the first reaction vessel.

7. The continuous method for preparing (mono- and dichloromethyl)benzenes according to any of claims 1 to 4, **characterised in that** it takes place in 2 reaction vessels.

8. The method according to claim 7, **characterised in that** it takes place with the introduction of fresh chlorine into the 2 reaction vessels.
